# FASCICULE DE BREVET EUROPEEN

(11) **EP 0 608 160 B1**
(45) Date de publication et mention de la délivrance du brevet: **23.12.1998**
(21) Numéro de dépôt: 94400067.8
(22) Date de dépôt: 12.01.1994
(51) Int. Cl.: C07C 231/02

(54) **Procédé de préparation d'acide maleamique**
Verfahren zur Herstellung von Maleinamidsäure
Process for the preparation of meleamic acid

(43) Date de publication de la demande: 27.07.1994
(73) Titulaire: RHODIA CHIMIE, 92408 Courbevoie Cédex (FR)
(72) Inventeur: Brun, Daniel W., F-69390 Charly (FR); Lahary, Pierre-Yves M., F-69006 Lyon (FR)
(74) Mandataire: Fabre, Madeleine-France

(56) Documents cités:
- DE-C- 945 987
- US-A- 2 459 964
- DATABASE WPI Section Ch, Week 7438, Derwent Publications Ltd., London, GB; Class E10, AN 74-66969V & JP-A-49 035 325 (NIPPON SHIKUBAI KAGAKU)
- DATABASE WPI Section Ch, Week 7335, Derwent Publications Ltd., London, GB; Class E10, AN 73-49652U & SU-A-362 821 (YU G MAMEDALIEV)
- CHEMICAL ABSTRACTS, vol. 79, no. 9, 1973, Columbus, Ohio, US; abstract no. 52751w, 'AMMONOLYSIS OF MALEIC ANHYDRIDE TO MALEIC ACID MONOAMIDE' page 319 ; & AZERB. KHIM. ZH. no. 4 , 1972 pages 13 - 15 BAGIROV, SH. T. ET AL

## Description

La présente invention a pour objet un procédé de préparation d'acide maléamique.

Classiquement l'acide maléamique est préparé à partir d'anhydride maléique et d'ammoniac. Les procédés correspondants connus mettent en oeuvre l'anhydride maléique soit sous une forme soluble ou sous sa forme vaporisée.

Le brevet JP 7 435 325 décrit plus particulièrement une voie de synthèse en milieu solvant organique à une température fluctuant entre la température ambiante et 100°C. L'ammoniac y est introduit sous forme gazeuse au sein du solvant. Une étape d'isolement subséquente conduit au produit attendu.

L'anhydride maléique, chauffée à au moins sa température de vaporisation c'est-à-dire à au moins 215°C, conduit également avec l'ammoniac gazeux à l'acide maléamique. Toutefois, cette réaction nécessite la présence d'un catalyseur. Il peut notamment s'agir d'un mélange de V2O5, P2O5 et Al2O3, (USSR 362821).

La présente invention a pour objet la mise au point d'une nouvelle voie de synthèse de l'acide maléamique plus économique que celles déjà existantes.

De manière inattendue, il a été mis en évidence que la réaction entre l'anhydride maléïque et l'ammoniac gazeux pouvait être réalisée efficacement en absence de tout solvant organique et de tout catalyseur. L'emploi de l'anhydride maléique sous une forme fondue permet de s'affranchir de la présence de ces deux composés.

Plus précisément la présente invention a pour objet un procédé de préparation de l'acide maléamique à partir de l'anhydride maléique et de l'ammoniac gazeux caractérisé en ce que l'anhydride maléïque se présente sous une forme fondue et est mis en contact intime avec de l'ammoniac gazeux.

Ce procédé a pour avantage de conduire directement à l'acide maléamique sans nécessiter la mise en oeuvre d'étapes de purification de type filtration, concentration, subséquentes. Ceci présente un grand intérêt tant sur le plan économique que sur celui de la mise en oeuvre à l'échelle industrielle.

Il ne nécessite pas en outre la présence annexe d'un solvant organique et/ou d'un catalyseur.

Selon le procédé selon l'invention, l'anhydride maléique est au préalable porté, dans un milieu de préférence anhydre et désoxygéné, à une température comprise entre environ sa température de fusion c'est à dire 50°C et 150°C et plus particulièrement entre 60 et 130°C. Il est clair que la fusion de l'anhydride maléique peut être obtenue par mise en oeuvre de tout moyen de chauffage quelconque.

Tout moyen technique susceptible de mettre en contact intime les deux composés peut être mis en oeuvre.

Les conditions opératoires favorables à une mise en contact intime entre les deux composants peuvent notamment être acquises grâce à une agitation vigoureuse du récipient contenant l'anhydride maléïque sous une forme fondue. Cette agitation de part la force centrifugeuse correspondante, disperse uniformément la masse de l'anhydride maléique fondue à la surface des parois du récipient en un film d'épaisseur très réduite. Il en résulte une surface importante de contact entre l'anhydride maléique ainsi dispersé et l'ammoniac injecté dans le récipient.

Il est clair qu'il est à la portée de l'homme de l'art de sélectionner et/ou d'adapter parmi le matériel déjà existant, le dispositif adéquat pour privilégier le contact intime entre les deux réactifs. Il peut s'agir de réacteurs agités, de sécheurs, d'atomiseurs....

La réaction entre l'anhydride maléique fondue et l'ammoniac est de préférence réalisée en milieu anhydre et à une température compatible avec la fusion dudit anhydride. Préférentiellement cette température se situe entre environ 50 et 150° et plus particulièrement entre 60 et 130°C.

L'ammoniac gazeux est, selon un mode de réalisation préféré de l'invention, introduit en continu à la surface de l'anhydride maléique fondu.

Dans le cadre de la présente invention, l'ammoniac gazeux est introduit seul ou en mélange avec un gaz inerte. Il peut en particulier s'agir d'argon ou d'azote. L'introduction de l'ammoniac en mélange avec un gaz inerte a notamment pour intérêt de permettre un meilleur contrôle de la température de réaction.

Selon un mode de réalisation particulier de l'invention, l'anhydride maléique, introduit dans un réacteur agité, au préalable désoxygéné, est porté à une température supérieure ou égale à sa température de fusion mais qui demeure inférieure à 150°C. De préférence, on applique un chauffage compris entre 60 et 130°C. L'anhydride maléïque étant sous forme fondue, on fait circuler au sein du récipient un courant d'ammoniac gazeux avec un flux contrôlé. Afin d'assurer la mise en contact intime entre l'anhydride maléïque et l'ammoniac, on procède à une agitation vigoureuse de préférence mécanique, du milieu réactionnel. L'évolution de la réaction est contrôlée à l'aide d'une sonde de température et d'un débitmètre pour NH₃. La durée de la réaction est bien entendu fonction du débit selon lequel est introduit la quantité d'ammoniac gazeux nécessaire à la conversion de l'anhydride maléïque en acide maléamique. A la fin de la réaction, on récupère directement l'acide maléamique attendu avec un rendement quasiment quantitatif et une pureté très satisfaisante.

L'acide maléamique ainsi obtenu est entre autre très intéressant à titre de produit de départ pour préparer le polysuccinimide et/ou l'acide polyaspartique.

L'exemple présenté ci-après à titre non limitatif de l'invention permettra de mettre en évidence d'autres avantages du procédé revendiqué.

### EXEMPLE:

6,4g d'anhydride maléïque solide sont introduits dans un réacteur en verre de forme tricol d'un volume de 100ml et muni d'un agitateur ancre raclant ainsi que d'une sonde de température. Après avoir purgé à l'azote, l'ensemble du dispositif le réacteur est introduit dans un bain d'huile préchauffé à 80°C. Lorsque l'anhydride maléique est fondu, on impose une forte agitation de manière à le répartir uniformément en une couche mince sur les parois du réacteur. Quant la température au sein du réacteur atteint 80°C on introduit un courant gazeux à température ambiante d'un mélange d'ammoniac et d'azote dans un rapport de 0,85 exprimé en volume. 1,11g d'ammoniac, correspondant à la stoechiométrie , sont ainsi introduits en 51 minutes.
On obtient 7,35 g d'un solide blanc soit avec un rendement de 97,9% qui possède les caractéristiques suivantes:
I.R.: 3383cm-1, 3208cm-1 (NH2); 1715 cm-1 (CO acide); 1618 cm-1(CO amide) Analyse thermique différentielle (10°C/min): 1 pic endothermique de fusion de 170°C.

## Revendications

1. Procédé de préparation d'acide maléamique à partir d'anhydride maléïque et d'ammoniac caractérisé en ce que l'anhydride maléique se présente sous une forme fondue et est mis en contact intime avec de l'ammoniac gazeux.

2. Procédé selon la revendication 1 caractérisé en ce que l'anhydride maléïque fondu et l'ammoniac gazeux sont mis en contact à une température comprise entre 50 et 150°C.

3. Procédé selon la revendication 2 caractérisé en ce que la température est comprise entre 60 et 130°C.

4. Procédé selon l'une quelconque des revendications précédentes caractérisé en ce que l'ammoniac gazeux est introduit sous la forme d'un mélange avec un gaz inerte.

5. Procédé selon la revendication 5 caractérisé en ce que le gaz inerte est l'azote.

6. Procédé selon l'une quelconque des revendications précédentes caractérisé en ce que l'opération de mise en contact est réalisée à l'aide d'un réacteur agité, d'un atomiseur ou d'un sécheur.

7. Procédé selon l'une quelconque des revendications précédentes caractérisé en ce que l'ammoniac gazeux est introduit en continu sur l'anhydride maléïque fondu.

8. Procédé selon la revendication 7 caractérisé en ce que la réaction entre l'anhydride maléïque fondu et l'ammoniac gazeux est réalisée dans un réacteur agité, sous des conditions d'agitation vigoureuses de manière à optimiser la surface de contact entre les deux composants.

## Patentansprüche

1. Verfahren zur Herstellung von Maleinamidsäure aus Maleinsäureanhydrid und Ammoniak, dadurch gekennzeichnet, daß das Maleinsäureanhydrid in geschmolzener Form vorliegt und mit dem gasförmigen Ammoniak in engen Kontakt gebracht wird.

2. Verfahren gemäß Anspruch 1, dadurch gekennzeichnet, daß das geschmolzene Maleinsäureanhydrid und der gasförmige Ammoniak bei einer Temperatur zwischen 50 und 150 °C in Kontakt gebracht werden.

3. Verfahren gemäß Anspruch 2, dadurch gekennzeichnet, daß die Temperatur zwischen 60 und 130 °C liegt.

4. Verfahren gemäß einem der vorhergehenden Ansprüche, dadurch gekennzeichnet, daß der gasförmige Ammoniak in Form eines Gemischs mit einem Inertgas eingeführt wird.

5. Verfahren gemäß Anspruch 4 dadurch gekennzeichnet, daß das Inertgas Stickstoff ist.

6. Verfahren gemäß einem der vorhergehenden Ansprüche, dadurch gekennzeichnet, daß der Vorgang des Inkontaktbringens mit Hilfe eines Rührreaktors, eines Zerstäubers oder eines Trockners durchgeführt wird.

7. Verfahren gemäß einem der vorhergehenden Ansprüche, dadurch gekennzeichnet, daß der gasförmige Ammoniak kontinuierlich über das geschmolzene Maleinsäureanhydrid eingeleitet wird.

8. Verfahren gemäß Anspruch 7, dadurch gekennzeichnet, daß die Reaktion zwischen dem geschmolzenen Maleinsäureanhydrid und dem gasförmigen Ammoniak in einem Rührreaktor unter heftigen Rührbedingungen, um die Kontaktfläche zwischen den beiden Bestandteilen zu optimieren, durchgeführt wird.

## Claims

1. Process for the preparation of maleamic acid from maleic anhydride and ammonia, characterized in that the maleic anhydride is in a molten form and is brought into close contact with gaseous ammonia.

2. Process according to Claim 1, characterized in that the molten maleic anhydride and the gaseous ammonia are brought into contact at a temperature of between 50 and 150°C.

3. Process according to Claim 2, characterized in that the temperature is between 60 and 130°C.

4. Process according to any one of the preceding claims, characterized in that the gaseous ammonia is introduced in the form of a mixture with an inert gas.

5. Process according to Claim 5, characterized in that the inert gas is nitrogen.

6. Process according to any one of the preceding claims, characterized in that the operation of bringing into contact is carried out by means of a stirred reactor, an atomizer or a dryer.

7. Process according to any one of the preceding claims, characterized in that the gaseous ammonia is introduced continuously onto the molten maleic anhydride.

8. Process according to Claim 7, characterized in that the reaction between the molten maleic anhydride and the gaseous ammonia is carried out in a stirred reactor, under vigorous agitation conditions such that the contact surface between the two components is optimized.
